# EUROPEAN PATENT SPECIFICATION

(11) **EP 1 004 305 B1**
(45) Date of publication and mention of the grant of the patent: **28.09.2011**
(21) Application number: 99913726.8
(22) Date of filing: 20.04.1999
(51) Int. Cl.: A61K 31/44, A61K 9/28, A61K 47/02, A61K 47/32, A61K 47/38

(54) **STABILIZED COMPOSITIONS CONTAINING BENZIMIDAZOLE-TYPE COMPOUNDS**
STABILISIERTE ZUSAMMENSTELLUNGEN DIE BENZIMIDAZOLE ENTHALTEN
COMPOSITIONS STABILISEES CONTENANT DES COMPOSES DU TYPE BENZIMIDAZOLE

(30) Priority: 20.04.1998 JP 10928898
(43) Date of publication of application: 31.05.2000
(73) Proprietor: Eisai R&D Management Co., Ltd., Tokyo 112-8088 (JP)
(72) Inventor: UKAI, Koji, Gifu-shi Gifu 500-8384 (JP); ICHIKAWA, Masaki, Tsuchiura-shi Ibaraki 300-0065 (JP); KATO, Takashi, Tsukuba-shi Ibaraki 305-0031 (JP); SUGAYA, Yukiko, Tsukuba-shi Ibaraki 305-0035 (JP); SUZUKI, Yasuyuki, Tsukuba-shi Ibaraki 305-0854 (JP); AOKI, Shigeru, Hashima-gun Gifu 501-6027 (JP); KATO, Akira, Tsukuba-shi Ibaraki 305-0035 (JP); KAWAMURA, Masao, Honjo-shi Saitama 367-0063 (JP); FUJIOKA, Satoshi, Ichinomiya-shi Aichi 491-0051 (JP)
(74) Representative: HOFFMANN EITLE
(86) International application number: PCT/JP1999/002098
(87) International publication number: WO 1999/053918

(56) References cited:
- EP-A- 0 761 212
- WO-A-96/01623
- WO-A-96/24338
- WO-A-99/48498
- WO-A1-92/22284
- GB-A- 2 189 698
- JP-A- 9 216 847
- JP-A- 9 511 257
- DRUG DEVELOPMENT AND INDUSTRIAL PHARMACY, Vol. 18, No. 13, 1992, TETSURO TABATA et al., "Stabilization of a New Antiulcer Drug (Lansoprazole) in the Solid Dosage Forms", p. 1437-1447, XP002921226

## Description

### Field of the Invention

The present invention relates to pharmaceutical preparations of the solid dosage form for internal use comprising rabeprazole sodium

### Prior Art

A benzimidazole type compound or an alkali metal salt thereof has a strong inhibitory action on the so-called proton pump, and it is widely used as a therapeutic agent for stomach ulcer, duodenal ulcer etc., by inhibiting gastric acid secretion. On the other hand, the benzimidazole type compound is chemically very unstable, so various measures have been invented for pharmaceutical manufacturing thereof. For example, JP-A 62-277322 discloses a process for producing a stabilized pharmaceutical composition comprising a basic inorganic salt of magnesium and/or calcium incorporated into a benzimidazole type compound, and JP-A 62-258320 discloses an oral pharmaceutical preparation prepared by incorporating an alkali compound into the portion of a core containing a benzimidazole type compound, then coating it with fillers for tablets soluble in water or rapidly degradable with water or with a polymeric and water-soluble film-forming compound, and further coating it with an enteric coating.

WO 96/01623 discloses a pharmaceutical preparation consisting of a core containing omeprazole or an alkaline salt thereof, which core may be coated with a separating layer, an enteric coating layer and an over-coating layer. The core is prepared from seeds coated with a layer of omeprazole, alkaline substances and further excipients.

WO 96/24338 discloses an oral pharmaceutical dosage form comprising a core material containing a benzimidazole-type proton pump inhibitor or an alkaline salt thereof, such as omeprazole, lansoprazole, pantoprazole, leminoprazole and pariprazole, one or more alkaline reacting compound(s) and optionally pharmaceutically acceptable excipients, wherein the core material is coated with a water soluble separating layer and an enteric coating layer.

GB-A-2 189 698 discloses an oral dosage form consisting of a core containing omeprazole or an alkaline salt thereof mixed with alkaline compounds, the core being coated with a separating layer and an enteric coating layer.

EP-A-0 761 212 discloses an effervescent composition comprising a core-shell powder. The core-shell powder consists of a fine granular core coated with a drug containing layer comprising a water-soluble polymer and an acid-sensitive drug substance, such as a benzimidazole compound, e.g. lansoprazole or omeprazole, and an enteric coating layer.

WO 99/48498 dislcoses an oral pharmaceutical formulation of a benzimidazole compound as drug substance, such as omeprazole, lansoprazole, timoprazole and pantoprazole, comprising a core covered by a drug layer, an intermediate coating layer and an enteric coating layer.

WO 92/22284 discloses an oral pharmaceutical composition containing pantoprazole, which consist of a core comprising pantoprazole and an anorganic base, an intermediate layer and an enteric outer layer.

WO 96/23500 discloses a pharmaceutical formulation containing a benzimidazol compound, such as omeprazole and lansoprazole. The formulation comprises a neutral core, a first layer containing the benzimidazol compound, a water-soluble polymer and non-alkaline reaction vehicles, a second isolating layer and an enteric layer.

JP 9216817 discloses a film coated preparation being moisture resistant and easily degradable with water containing a three-layer coating consisting of an inner coating with a water soluble polymer, a wax coating and an outer coating with a water soluble polymer.

The effects of adding magnesium carbonate as an alkaline stabilizer to enteric granules of lansoprazole were examined by Tabatar et al. (T. Tabatar, T. Makino, T. Kashihara, S. Hirai, N. Kitamori, H. Toguchi "Stabilization of a new antiulcer drug (Lansoprazole) in the solid dosage forms", Drug Development and Industrial Pharmacy, Vol. 18, Issue 13, 1992, pages 1437-1447).

However, the stability of such pharmaceutical preparations is still insufficient even by the prior art described above, so there is demand for further improvements. That is, the object of the present invention is to further stabilize a pharmaceutical preparation of the solid dosage form for internal use comprising a benzimidazole type compound.

### Disclosure of the Invention

The present invention relates to a composition comprising a core and an enteric coating, wherein the core comprises 1 part by weight of rabeprazole sodium, 0.5 to 5 parts by weight of crospovidone and 0.01 to 2 parts by weight of sodium hydroxide.

Further, the present invention relates to a pharmaceutical preparation comprising a core as defined above which is coated with an entaric coating.

Further, the present invention relates to a pharmaceutical preparation comprising a core as defined above coated with an intermediate coating and further with an enteric coating. The present invention further relates to a pharmaceutical preparation comprising a core as defined above coated with an intermediate coating, further with an enteric coating and then with a moisture resistant coating.

The present invention relates to a pharmaceutical composition comprising (A) rabeprazole sodium and (B) and crospovidone.

Further, the present invention relates to a pharmaceutical preparation comprising a core consisting of the composition described above and an enteric coating. The pharmaceutical preparation may comprise an intermediate coating, an enteric coating and a moisture resistant coating besides the core.

The moisture resistant coating is effective not only for the benzimidazole type compound but also for a drug whose decomposition is observed to be accelerated both in the presence of water and upon contact with gastric acid. The present invention relates to a pharmaceutical preparation comprising a core coated with an enteric coating and further with a moisture resistant coating, said core being defined as above.

In the present invention, the benzimidazole type compounds is rabeprazole sodium. The scructural formula of rebeprazole is shown in formula 3.

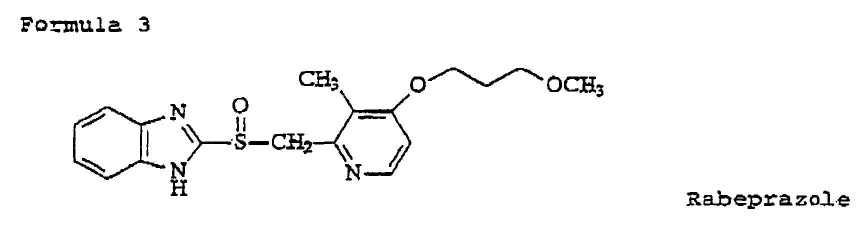

Hereinafter, the benzimidazole type compound or an alkali metal salt thereof is collectively referred to as benzimidazole type compound.

The benzimidazole type compound can be produced in a known method. For example, the compound can be produced by any methods disclosed in JP-A 52-62275, JP-A 54-141783, JP-A 1-6270 etc.

Sodium carbonate, potassium carbonate, sodium hydroxide, potassium hydroxide and hydroxypropyl cellulose in the present invention are mentioned in the Japanese Pharmacopoeia, and these are commercially available and easily obtainable. Aminoalkyl methaacrylate copolymer E, which is mentioned in the standards of non-medicines in the Japanese Pharmacopoeia, can be easily obtained. Further, crospovidone is a substance mentioned in the standards of pharmaceutical additives, and its commercial products of various grades with varying particle diameters are easily available, and their particle diameters can be regulated as necessary by a grinding device such as hammer mill.

The blending ratio of the rabeprazole sodium to sodium hydroxide, is 0.01 to 20 parts by weight, preferably 0.01 to 10 parts by weight, more preferably 0.1 to 10 parts by weight in total, to 1 part by weight of rabeprazole sodium. In the present invention, sodium carbonate, potassium carbonate, potassium hydroxide, aminoalkyl methaacrylate copolymer E, arginine aspartate, hydroxypropyl cellulose and crospovidone can be used in addition to sodium hydroxide. It is more effective to incorporate 1) crospovidone and 2) sodium hydroxide alone or together with potassium hydroxide or sodium carbonate into rabeprazole sodium. The blending ratio of crospovidone is 0.5 to 5 parts by weigh, and the ratio of sodium hydroxide is 0.01 to 2 parts by weight, to 1 part by weight of rabeprazole sodium.

A benzimidazole type compound when decomposed during storage under heating and humid conditions is observed to undergo significant coloring changes in particular. The composition and/or the pharmaceutical preparation of the invention comprising the above-described various additives incorporated into it possesses the particularly outstanding effect of not only improving the stability of the ingredients but also inhibiting the coloring changes.

Conventionally used excipients such as lactose and mannitol can be used to prepare a pharmaceutical preparation by use of the invented composition as defined above. Preferably, hydroxypropyl cellulose is used as a binder and crospovidone is used as a disintegrating agent.

It is known that crospovidone used generally as a disintegrating agent, when finely ground, can reduce the disintegrating force and swelling force inherent in the original disintegratins agent. Finely ground crospovidone having small particle diameters is used as a stabilizer for the benzimidazole type compound in the present invention, and it can be added in a larger amount than the amount of a usual disintegrating agent (usually 10 % or less). The average particle diameter of crospovidone is several µm to 50 µm, more preferably 4 µm to 50 µm.

Accordingly, the crospovidone used in the composition or in the pharmaceutical preparation according to the present invention is preferably crospovidone having small average particle diameters of several µm to 50 µm, preferably 4 µm to 50 µm. As a matter of course, finely ground crospovidone and usual crospovidone may be used in combination.

The crospovidone, though varying depending on manufacturer and lot number, often contains a slight amount of peroxides as impurities. Rabeprazole is inherently liable to oxidation so that when blended along with crospovidone, it may contain an antioxidant.

The antioxidant includes, but is not limited to, sodium sulfite, sodium pyrosulfite, vitamin E, rongalite, thioglycerol, sodium thiosulfate, ascorbate and acetyl cysteine.

Further, the present invention relates to a pharmaceutical preparation comprising a core as defined above coated with an enteric coating. In the present invention, the term "core" refers to tablets, granules etc. Further, the present invention encompasses a pharmaceutical preparation comprising a core as defined above coated with an enteric coating, said core being coated thereon with spherical granules consisting, as seed granules, of refined white sugar, a mixture of white sugar and starch, or crystalline cellulose etc. rabeprazole sodium is very unstable under acidic conditions, so when administered, it is decomposed immediately in contact with gastric acid in the stomach, to lose its physiological activity. Accordingly, it should be formed as a pharmaceutical preparation not dissolved in the stomach, that is, a pharmaceutical preparation having rabeprazole sodium containing core coated with an enteric substance in order to prevent it from being decomposed in the stomach.

Further, the present invention relates to pharmaceutical preparation comprising a core as defined above coated with an intermediate coating and further with an enteric coating core comprising. Since the enteric coating is made generally of an acidic substance, its direct contact with a benzimidazole type compound is not preferable. Accordingly, an inert intermediate coating can be provided between the core comprising rabeprazole sodium and the enteric coating. The term "inert" refers to a substance not adversely affecting the stability of the benzimidazole type compound. The inert intermediate coating may be made of a water-soluble polymer, a water-soluble or water-disintegrating substance or a water-insoluble substance, and specific examples include hydroxypropyl cellulose, hydroxypropylmethyl cellulose, aminoalkyl methaacrylate copolymer E, lactose, mannitol, starch, crystalline cellulose, ethyl cellulose, vinyl acetate etc. When an intermediate coating made of a water-insoluble substance is applied, water-insoluble fine particles may be mixed in the coating, as disclosed in JP-A 1-290628.

In the present invention, the above-described pharmaceutical preparation coated with an enteric coating may be coated with a moisture resistant coating. The moisture resistant coating is a coating for inhibiting the passage of steam, and it is functionally a coating which in itself inhibits the transmission of steam or a coating which captures steam in the coating to inhibit the inflow of steam into the inside.

The moisture resistant coating possesses the function of defending the preparation against invasion of water into the benzimidazole type compound to improve its stability while preventing the cracking and deformation of tablets originating from the swelling of finely ground crospovidone upon moisture absorption.

The moisture resistant coating may be either a water-soluble coating or a water-insoluble coating, and this coating includes, but is not limited to, a coating consisting of e.g. polyvinyl acetal diethyl aminoacetate, HA Sankyo (a mixture of polyvinyl acetal diethyl aminoacetate, hydroxypropylmethyl cellulose, stearic acid and fumaric acid) and/or polyvinyl alcohol etc., a coating comprising at least one of cellulose derivatives such as hydroxypropyl cellulose, hydroxypropylmethyl cellulose and ethyl cellulose incorporated into it, and/or a sugar coating based on white sugar.

The moisture resistant coating is useful not only for the benzimidazole type compound but also for a pharmaceutical preparation containing a drug having similar chemical properties. That is, its effect is observed to be significant when it is applied onto a pharmaceutical preparation containing a drug whose decomposition is observed to be accelerated both in the presence of water and upon contact with gastric acid.

The effect is particularly outstanding where the benzimidazole type compound is rabeprazole sodium as according to the present invention

Further, the present invention relates to a composition comprising 1) crospovidone and 2) sodium hydroxide alone or together with potassium into rabeprazole sodium.

As described above, the crospovidone used is preferably finely ground until its average particle diameter is decreased to several µm to 50 µm. Further, an antioxidant may be added to prevent the influence of trace peroxides contained in crospovidone, as described above. Accordingly, an antioxidant may be incorporated into the composition comprising 1) crospovidone and 2) sodium hydxoxide alone or together with or sodium incorporated into rabeprazole sodium.

The present invention relates further to a pharmaceutical preparation comprising a core which comprises 1) crospovidone and 2) sodium hydroxide alone or together with potassium hydroxide and/or sodium incorporated into rabeprazole sodium coated with an enteric coating.

The present invention relates further to a pharmaceutical preparation comprising a core which comprises 1) crospovidone and 2) sodium hydroxide along or together with potassium hydroxide or sodium carbonate incorporated into rabeprazole sodium coated with an intermediate coating and further with an enteric coating.

The present invention relates further to a pharmaceutical preparation comprising a core which comprises 1) crospovidone and 2) sodium hydroxide alone or together with sodium carbonate incorporated into rabeprazole sodium coated with an intermediate coating, further with an enteric coating and then with a moisture resistant coating.

The composition or the pharmaceutical preparation according to the present invention can be produced by any conventionally used processes.

For example, sodium hydroxide, and crospovidone is incorporated into rabeprazole sodium, then excipients are added thereto, and the mixture grantlated in a dry or wet granulating process, followed by adding a disintegrating agent such as crospovidone as necessary and subsequently tablatting the granules whereby the composition or the pharmaceutical preparation of the invention can be produced. Alternatively, for example, sodium hydroxide, and crospovidone is incorporated at high density into a rabeprazole sodium to prepare benzimidazole-containing granules, while placebo granules not containing the benzimidazole type compound are separately prepared, and then both the granules are mixed followed by adding a disintegrating agent such as crospovidone as necessary and subsequently tabletting the granules. As a matter of course, these processes are non-limiting examples.

In a concrete reference example, e.g. 100 g sodium rabeprazole as the benzimidazole type compound, 30 g sodium carbonate and 130 g mannitol are mixed, and hydroxypropyl cellulose dissolved in ethanol is gradually added to the mixture under stirring, followed by granulation, drying and screening through a 24-mesh screen. 30 g crospovidone and 2 g calcium stearate are added thereto, mixed and tabletted whereby tablets each weighing 135 mg can be obtained.

These tablets are sprayed by using a fluidized-bed granulator with a solution of hydroxypropyl cellulose in ethanol and further with a solution of hydroxypropylmethyl cellulose phthalate or an enteric methaacrylate copolymer in water/ethanol whereby enteric tablets provided with an intermediate coating can be produced.

According to the present invention, it is possible to stabilize the very unstable rabeprazole sodium. Examples of this effect are shown below.

### Experimental Examples

50 mg sodium rabeprazole and 450 mg additives shown in the table below were mixed in a mortar.

The mixture was introduced into a transparent glass vial and stored in a cold place or at 60 °C or 40 °C under 75 % relative humidity for 1 week and their content was determined by high performance liquid chromatography. Assuming that the content of the sample stored in the cold place is 100 %, the degrees of the residual content under the respective conditions are shown in Tables 1 through 3. Further, their coloring changes were visually evaluated. The sodium rabeprazole used was amorphous in Table 1 and crystalline in Tables 2 and 3. In Table 1, low-substituted hydroxypropyl cellulose (expressed as L-HPC) used as a disintegrating agent in addition to amorphous sodium rabeprazole was blended in the control; in Table 2, a sample further incorporating aluminum hydroxide (expressed as Al(OH)₃ in the table) i.e. an alkaline inorganic salt used as an antacid agent was used; and in Table 3, a sample incorporating polyvinyl pyrrolidone (expressed as PVP in the table) was used as a binder.

**Table 1 Compatibility Test of Sodium Rabeprazole**

| | | 60°C | 40°C-75%RH |
|---|---|---|---|
| Control | sodium rabeprazole (amorphous) | 99.1 | 93.9 |
| | sodium rabeprazole + L-HPC | 80.4 | 73.3 |
| The present application | sodium rabeprazole + crospovidone | 98.1 | 90.4 |
| Unit : % | | | |

**Table 2 Compatibility Test of (crystalline) Sodium Rabeprazole**

| | | 60°C | 40°C-75%RH |
|---|---|---|---|
| Control | sodium rabeprazole (crystalline) | 99.8 | 91.8 |
| | sodium rabeprazole + L-HPC | 62.2 | 75.0 |
| | sodium rabeprazole + Al(OH)₃ | 36.9 | 26.2 |
| The present application | sodium rabeprazole + crospovidone | 93.3 | 89.5 |
| | sodium rabeprazole + Na₂CO₃ | 99.1 | 90.3 |
| | sodium rabeprazole + Arg · Asp | 97.5 | 90.7 |
| Unit : % | | | |

**Table 3 Compatibility Test of (Crystalline) Sodium Rabeprazole**

| | | 60°C | 40° C.75%RH |
|---|---|---|---|
| Control | sodium rabeprazole (crystalline) | 97.3 | 86.9 |
| | sodium rabeprazole + PVP | 89.5 | 67.7 |
| | sodium rabeprazole + hydroxypropyl cellulose (Ref.) | 92.0 | 86.9 |
| | sodium rabeprazole Na₂CO₃ (Réf.) | 93.0 | 82.8 |
| | sodium rabeprazole + NaOH | 91.6 | 99.8 |
| | sodium rabeprazole + KOH (Ref.) 92.6 | | 95.8 |
| | sodium rabeprazola + Eudragit E (Ref.) | 102.4 | 86.0 |
| | sodium rabeprazole + K₂CO₃ (Ref.) | 104.5 | 81.3 |
| Unit : % | | | |

Any coloring changes of the blended samples according to the present invention were lower than those of the controls. Further, it is evident from the results of content stability in Tables 1 through 3 that the ingredients used that is, sodium carbonate (as reference substance expressed as Na₂CO₃ in the table), potassium carbonate (as reference substance expressed as K₂CO₃ in the table), sodium hydroxide (expressed as NaOH in the table), potassium hydroxide (as reference substance expressed as KOH), aminoalkyl methaacrylate copolymer E (as reference substance expressed as Eudragit E^{®}), arginine aspartate (as reference substance expressed as Arg Asp in the table), hydroxypropyl cellulose (as reference substance) and crospovidone stabilize the benzimidazole type compound. Effect of Sodium Carbonate as reference substance in Tablets

Tablets containing different amounts of sodium carbonate, obtained in Examples 4 to 9 shown below, were stored at 40 °C under 75 % relative humidity for 1 week, and the contents of sodium rabeprazole in the tablets as determined by high performance liquid chromatography were shown in Table 4.

**Table 4**

| Stability Evaluation of Tablet Formulations by Wet Granulation | | | | | | |
|---|---|---|---|---|---|---|
| Formulation | Ref. Ex.4 | Ref. Ex.5 | Ref. Ex.6 | Ref. Ex.7 | Ref. Ex.8 | Ref. Ex.9 |
| (1week) | | | | | | |
| cold place | 99.4 | 99.0 | 98.7 | 99.4 | 99.5 | 98.9 |
| 40°C-75%RR | 83.8 | 85.7 | 85.1 | 92.5 | 92.8 | 95.5 |

| (1month) | | | | | | |
|---|---|---|---|---|---|---|
| cold place | 99.7 | 99.7 | 99.7 | 99.7 | 99.7 | 99.6 |
| 2°C-75%RB | 97.8 | 98.5 | 98.3 | 99.2 | 99.3 | 99.3 |

| | | | | | | |
|---|---|---|---|---|---|---|
| Unit : % | | | | | | |

Because the stability of the content of sodium rabeprazole in the tablets is improved depending on the amount of sodium carbonate added, the effect of sodium carbonate added is evident.

### Effect of Crospovidone in Tablets

Tablets containing different amounts of crospovidone powder, obtained in Examples 10 to 12 shown below, were stored at 40°C under 75% relative humidity for 1 week, and the contents of sodium rabeprazole in the tablets as determined by high performance liquid chromatography were shown in Table 5. The tablets were subject to less coloring change as the amount of the crospovidone powder added was increased.

**Table 5**

| Stabilize of Crospovidone-Added Tablets by Wet Granulation | | | |
|---|---|---|---|
| formulation | Ref. Ex.10 | Ref. Ex.11 | Ref. Ex.12 |
| (1week) | | | |
| cold place | 99.7 | 99.7 | 99.7 |
| 40°C-75%RH | 97.8 | 98.5 | 98.3 |

| (1month) | | | |
|---|---|---|---|
| cold place | 99.4 | 99.0 | 98.7 |
| 40°C-75%RH | 83.8 | 85.7 | 85.1 |

| | | | |
|---|---|---|---|
| Unit : % | | | |

It is evident that the stability of the Rabeprazole sodium is improved by adding crospovidone.

### Effect of Finely Ground Crospovidone in Tablets

Tablets containing crospovidone having a different average particle diameter, obtained in Reference Examples 16 to 18 shown below, were scored in a cold place or at 25°C under 75 % relative humidity for 1 month and then evaluated for their thickness to evaluate the ratio of swelling of the tablets stored at 25 °C under 75 % relative humidity to swelling of the tablets stored in the cold place. The results were that the ratios of swelling of the tablets containing crospovidone having average particle diameters of 51 µm, 12 µm and 6 µm were 1.61, 1.48 and 1.43, respectively.

The smaller the average of the particle diameter of the crospovidone was, the smaller the ratio of the swelling of the tablets became. Therefore, as crospovidone is made fine powder having a small average particle diameter, the cracking or deformation resulting from the swelling of the tablets is reduced. Accordingly, it is evident that the particle size reduction of crospovidone contributes to improvement of stability of tablets. Effect of a Moisture Resistant Coating Applied onto Tablets Coated with an Enteric Coating

Tablets coated with an enteric coating and tablets coated with both an enteric coating and a moisture resistant coating, obtained in Examples 19 to 20 shown below, were stored at 25 °C under 75 % relative humidity for 1 week, and the content of a rabeprazole analogue (impurities) in the tablets was determined by high performance liquid chromatography. The results indicated that the contents of the rabeprazole analogue (impurities) in the tablets coated with an enteric coating and the tablets coated with both an enteric coating and a moisture resistant coating were 2.38 % and 2.23 %, respectively.

It is evident that the tablets coated with both an enteric coating and a moisture resistant coating possess stability equal to or higher than that of the tablets coated with an enteric coating.

Placebo tablets obtained in Examples 21 to 23 shown below were stored in a cold place or at 40°C under 75 % relative humidity for 1 week and then evaluated for their thickness to evaluate the ratio of swelling of the tablets stored at 40°C under 75 % relative humidity to swelling of the tablets stored in the cold place. The results indicated that the ratios of swelling of the tablets coated with an enteric coating, tablets prepared by coating said enteric coating-coated tablets with a moisture resistant coating, and tablets prepared by coating said enteric coating-coated tablets with a moisture resistant coating consisting of HA (Sankyo) (i.e., a mixture of polyvinyl acetal diethyl aminoacetate, hydroxypropylmethyl cellulose, macrogol and talc) were 1.15, 1.03 and 1.12, respectively.

Since the degree of swelling of the tablets coated with both an enteric coating and a moisture resistant coating is smaller during storage than that of the tablets coated with an enteric coating only, it is evident that the stability in shape of the tablets is improved.

Effect of an Antioxidant Added to the Portion of a Core Containing rabeprazole sodium

Tablets containing a different amount of a peroxide, obtained in Examples 24 to 26 shown below, were measured for the content of a sodium rabeprazole analogue (impurities) by high performance liquid chromatography. The results indicate that the amounts of the initial rabeprazole analogue in the tablets incorporating crospovidone containing 18 ppm, 190 ppm and 310 ppm peroxide were 0.65 %, 0.88 % and 1.13 % respectively, indicating that as the amount of the peroxide in crospovidone is increased, the decomposition of sodium rabeprazole is promoted to increase the amount of the analogue.

Further, 1g crospovidone containing 201 ppm peroxide was accurately taken, and sodium sulfite (amounts: 4 levels i.e. no addition, 0.02 %, 0.05 % and 0.10 %) was added thereto and mixed well, and the amount of the peroxide in the mixture was determined according to a test method described in the Japanese Pharmacopoeia. The results indicated that the amounts of the peroxide in the compositions wherein the amounts of sodium sulfite added were none, 0.02 %, 0.05 % and 0.10 %, were 201 ppm, 184 ppm, 108 ppm, and 0 ppm respectively, indicating that as the amount of sodium sulfite added was increased, the amount of the peroxide was reduced.

From the foregoing, it is evident that the stability of the benzimidazole type compound in a pharmaceutical preparation is improved by adding the antioxidant to the portion of cores in tablets containing rabeprazole sodium and crospovidone.

### Examples

Hereinafter, the present invention is described more in detail by reference to Examples.

### Reference Example 1 :

10 g sodium carbonate and 100 g mannitol were added to and mixed with 10 g sodium rabeprazole, and 2.5 g hydroxypropyl cellulose dissolved in ethanol was gradually added to the mixture under stirring to make granules which were dried and screened followed by adding calcium stearate and tabletting to give tablets each weighing 120 mg containing 10 mg sodium rabeprazole.

### Reference Example 2 :

The tablets obtained in Example 1 were sprayed by using a fluidized-bed granulator with a solution of 10 g hydroxypropylmethyl cellulose phthalate dissolved in a mixed solvent of water and ethanol (2 : 8), to produce enteric tablets.

### Reference Example 3

The tablets obtained in Example 1 were sprayed by using a fluidized-bed granulator with a solution of hydroxypropylmethyl cellulose in ethanol, to produce enteric tablets in the same manner as in Example 2.

### Reference Examples 4 to 9

0 to 10 g sodium carbonate and 15 to 90 g mannitol were added to and mixed with 10 g sodium rabeprazole, and 0.7 to 2 g hydroxypropyl cellulose dissolved in ethanol was gradually added to the mixture to make granules under stirring in a wet granulation process, thus preparing the granules containing sodium rabeprazole. Separately, 2 g hydroxypropyl cellulose dissolved in ethanol was gradually added to 100 g mannitol to produce granules under stirring in a wet process to prepare placebo granules. Then, the main-drug granules were mixed with the placebo granules, and 5 % crospovidone and a slight amount of magnesium stearate were added thereto in a powdery form and tabletted to give tablets each weighing 100.5 mg containing 10 mg sodium rabeprazole. Each formulation is shown in Table 6.

**Table 6 Tablet Formation by Wet Granulation**

| | Formulation | Ref. Ex.4 | Ref. Ex.5 | Ref. Ex.6 | Ref. Ex.7 | Ref. Ex.8 | Ref. Ex.9 |
|---|---|---|---|---|---|---|---|
| Active granule | sodium rabeprazole | 10.0 | 10.0 | 10.0 | 10.0 | 10.0 | 10.0 |
| | anhydrous sodium carbonate | - | - | - | 5.0 | 5.0 | 10.0 |
| | mannitol | 82.0 | 30.0 | 20.0 | 25.0 | 15.0 | 20.0 |
| | hydroxypropyl cellulose | 2.0 | 1.0 | 0.7 | 1.0 | 0.7 | 1.0 |
| | (sub-total) | 94.0 | 41.0 | 30.7 | 41.0 | 30.7 | 41.0 |
| Placebo granule | mannitol | - | 52.0 | 62.1 | 52.0 | 62.1 | 52.0 |
| | hydroxypropyl cellulose | - | 1.0 | 1.2 | 1.0 | 1.2 | 1.0 |
| | (sub-total) | 0.0 | 53.0 | 63.3 | 53.0 | 63.3 | 53.0 |
| Powder added | crospovidone | 5.0 | 5.0 | 5.0 | 5.0 | 5.0 | 5.0 |
| | magnesium stearate | 1.5 | 1.5 | 1.5 | 1.5 | 1.5 | 1.5 |
| | (sub-total) | 6.5 | 6.5 | 6.5 | 6.5 | 6.5 | 6.5 |
| | total | 100.5 | 100.5 | 100.5 | 100.5 | 100.5 | 100.5 |
| Unit : mg | | | | | | | |

### Reference Examples 10 to 12

Tablets were obtained in the same manner as in Examples 4 to 9 except that the amounts of crospovidone powder added were 3 levels, that is, 0, 2.5, and 5 %. Each formulation is shown in Table 7.

**Table 7**

| Formulation of Crospovidone-Added Tablets by Wet Granulation | | | | |
|---|---|---|---|---|
| | Formulation | Ref. Ex.10 | Ref. Ex.11 | Ref Ex.12 |
| Active granule | sodium rabeprazole (crystalline) | 10.0 | 10.0 | 10.0 |
| | anhydrous sodium carbonate | 5.0 | 5.0 | 5.0 |
| | mannitol hydroxypropyl cellulose | 25.0 1.0 | 25.0 1.0 | 25.0 1.0 |
| | (sub-total) | 41.0 | 41.0 | 41.0 |
| Placebo granule | mannitol | 56.9 | 54.4 | 52.0 |
| | hydroxypropyl cellulose | 1.1 | | 1.1 1.0 |
| | (sub-total) | 58.0 | 55.5 | 53.0 |
| Powder added | crospovidone | - | 2.5 | 5.0 |
| | magnesium stearate | 1.5 | 1.5 | 1.5 |
| | (sub-total) | 1.5 | 4.0 | 6.5 |
| | total | 100.5 | 100.5 | 100.5 |
| Unit : mg | | | | |

### Reference Examples 13 to 14

According to the 2 formulations shown in Table 8, 0 to 50 g sodium carbonate, 79.3 to 84.3 g mannitol, 4.2 g crospovidone and 1.5 g magnesium stearate were added to 10 mg sodium rabeprazole, mixed well, and directly tabletted to give tablets each weighing 100 mg containing 10 mg sodium rabeprazole.

**Table 8**

| Tablet Formulation by Direct Tabletting | | |
|---|---|---|
| Formulation | Ref. Ex.13 | Ref. Ex.14 |
| sodium rabeprazole (crystalline) | 10.0 | 10.0 |
| anhydrous sodium carbonate. | | 5.0 |
| mannitol | 84.3 | 79.3 |
| crospovidone | 4.2 | 4.2 |
| magnesium stearate | 1.5 | 1.5 |
| total | 100.0 | 100.0 |
| Unit : mg | | |

### Reference Example 15

50 g sodium carbonate and 2 g magnesium stearate were added to 100 g sodium rabeprazole, mixed well to make granules under dry compression granulation process, to prepare main-drug granules. Separately, 76.3 g mannitol was added to and mixed well with 4.2 g crospovidone, and 2.3 g hydroxypropyl cellulose dissolved in ethanol was gradually added thereto to make granules under stirring in a wet process to prepare placebo granules. Then, the main-drug granules were mixed with the placebo granules, and a slight amount of magnesium stearate was added thereto in a powdery form and tabletted to give tablets each weighing 100 mg containing 10 mg sodium rabeprazole as shown in Table 9.

**Table 9**

| Tablet Formulation by Dry Granulation | | |
|---|---|---|
| | Formulation | Ref. Ex.15 |
| Active granule | sodium rabeprazole (crystalline) | 10.0 |
| | anhydrous sodium carbonate | 5.0 |
| | magnesium stearate | 0.2 |
| | (sub-total) | 15.2 |
| Placebo granule | mannitol | 76.8 |
| | crospovidone | 4.2 |
| | hyroxypropyl cellulose | 2.3 |
| | (sub-total) | 83.3 |
| added | Powder magnesium stearate | 1.5 |
| | total | 100.0 |
| Unit : mg | | |

### Reference Examples 16 to 18

527 g crospovidone having a different average particle diameter and 20 g hydroxypropyl cellulose were mixed with 100 g sodium rabeprazole, and 3 g magnesium stearate was added thereto in a powdery form, followed by tabletting to give tablets each weighing 65 mg containing 10 mg sodium rabeprazole as shown in Table 10. Crospovidone used is a product of BASF Ltd., and its average diameter is 51 µm for Colidone CL^{™}, 12 µm for Colidone CLM^{™} and 6 µm for a hammer mill-ground product of Colidone CLM^{™}.

**Table 10**

| Formulations Containing Crospovidone having Different Particle Diameters | | | |
|---|---|---|---|
| Formulation | Ref. Ex.16 | Ref. Ex.17 | Ref Ex.18 |
| sodium rabeprazole | 10.0 | 10.0 | 10.0 |
| crospovidone (colidone CL) | 52.7 | - | - |
| crospovidone (colidone CLM) | - | 52.7 | - |
| crospovidone (ground product of colidone CLM) | - | - | 52.7 |
| hydroxypropyl cellulose | 2.0 | 2.0 | 2.0 |
| magnesium stearate | 0.3 | 0.3 | 0.3 |
| (sub-total) | 65.0 | 65.0 | 65.0 |
| Unit : mg | | | |

| | | | |
|---|---|---|---|
| Note : Average diameters Crospovidone (Colidone CL): 51 µm Crospovidone (Colidone CLM): 12 ^{µ}m Crospovidone (ground product of Colidone.CLM): 6 ^{µ}m Examples 19 to 20 | | | |

The portion of a core containing sodium rabeprazole was granulated with ethanol and coated with a water-insoluble intermediate coating containing ethyl cellulose, crospovidone and magnesium stearate. Further, the resulting granules were coated with a coating to give tablets coated with an enteric coating or with both an enteric coating and a moisture resistant coating. The formulation is shown in Table 11.

**Table 11**

| Formulation of a Pharmaceutical Preparation Having an Enteric Coating and a Moisture Resistant Coating Applied Thereon | | | |
|---|---|---|---|
| | Formulation | Ex.19 | Ex.20 |
| Core | sodium rabeprazole | 10.0 | 10.0 |
| | mannitol | 36.2 | 36.2 |
| | crospovidone | 15.6 | 15.6 |
| | sodium hydroxide | 0.1 | 0.1 |
| | anhydrous sodium carbonate | 5.0 | 5.0 |
| | hydroxypropyl cellulose | 2.0 | 2.0 |
| | magnesium stearate | 1.1 | 1.1 |
| | (sub-total) | 70.0 | 70.0 |
| Intermediate coating | ethyl cellulose | 0.5 | 0.5 |
| | crospovidone | 1.0 | 1.0 |
| | magnesium stearate | 0.1 | 0.1 |
| | (sub-total) | 1.6 | 1.6 |
| Enteric coating | hydroxypropyl cellulose cellulose phthalate | 8.0 | 8.0 |
| | monoglyceride | 0.8 | 0.8 |
| | talc | 0.75 | 0.75 |
| | titanium oxide | 0.4 | 0.4 |
| | yellow iron oxide | 0.05 | 0.05 |
| | (sub-total) | 10.0 | 10.0 |
| Moisture resistant coating | hydroxypropylmethyl cellulose | - | 3.0 |
| | macrogol | - | 0.6 |
| | talc | - | 1.4 |
| | (sub-total) | | 5.0 |
| total | | 81.6 | 86.6 |
| Unit : mg | | | |

### Examples 21 to 23

As placebo tablets not containing the benzimidazole type compound, tablets having a water-soluble intermediate layer of hydroxypropyl cellulose applied onto the portion of cores therein were prepared. The tablets were coated further with an enteric coating to prepare tablets coated with an enteric coating, and further the enteric coating-coated tablets were sprayed with white sugar or HA (Sankyo) to prepare tablets coated with a moisture resistant coating. The formulation is shown in Table 12.

**Table 12**

| Placebo Formulation | | | | |
|---|---|---|---|---|
| | Formulation | Ex.21 | Ex.22 | Ex.23 |
| Core | mannitol | 31.8 | 31.8 | 31.8 |
| | crospovidone (colidone CALM) | 27.7 | 27.7 | 27.7 |
| | hydroxypropyl cellulose | 5.0 | 5.0 | 5.0 |
| | magnesium stearate | 0.5 | 0.5 | 0.5 |
| | (sub-total) | 65.0 | 65.0 | 65.0 |
| Intermediate coating | hydroxypropyl cellulose | 3.0 | 3.0 | 3.0 |
| Enteric coating | hydroxypropylmethyl cellulose phthalate | 8.0 | 8.0 | 8.0 |
| | monoglyceride | 0.8 | 0.8 | 0.8 |
| | talc | 0.75 | 0.75 | 0.75 |
| | titanium oxide | 0.4 | 0.4 | 0.4 |
| | yellow iron oxide | 0.05 | 0.03 | 0.05 |
| | (sub-total) | 10.0 | 10.0 | 10.0 |
| Moisture resistant coating | white sugar | - | 10.0 | - |
| | HA (Sankyol)* | - | - | 10.0 |
| total | | 78.0 | 88.0 | 88.0 |
| Unit : mg | | | | |

| | | | | |
|---|---|---|---|---|
| Note: HA (Sankyo)* | | | | |

A mixture of polyvinyl acetal diethyl aminoacetate, hydroxypropylmethyl cellulose, Macrogol and talc. Reference Examples 24 to 26

Tablets containing crospovidone with different contents of sodium rabeprazole and a peroxide, sodium hydroxide and sodium carbonate were obtained by granulation in a wet process, according to the formulation in Table 13.

**Table 13**

| Formulation Containing Crospovidone with Different Contents of Peroxide | | | |
|---|---|---|---|
| Formulation | Ex.24 | Ex.25 | Ex.26 |
| sodium rabeprazole | 10.0 | 10.0 | 10.0 |
| mannitol | 36.9 | 36.9 | 36.9 |
| crospovidone (INF-10)*1 | 14.0 | - | - |
| crospovidone (INF-10)*2 | - | 14.0 | - |
| crospovidone (colidone CLM)*3 | - | - | 14.0 |
| crospovidone (colidone CL) | 14.0 | 14.0 | 14.0 |
| sodium hydroxide | 0.5 | 0.5 | 0.5 |
| anhydrous sodium carbonate | 2.5 | 2.5 | 2.5 |
| hydroxypropyl cellulose | 2.0 | 2.0 | 2.0 |
| magnesium stearate | 1.1 | 1.1 | 1.1 |
| (total) | 70.0 | 70.0 | 70.0 |
| Unit:mg | | | |

| | | | |
|---|---|---|---|
| Note: Crospovidone (INF-10)*1: (peroxide content: 18 ppm) Crospovidone (INF-10)*2: (peroxide content: 190 ppm) Crospovidone (Colidone CLM) *3: (peroxide content: 310 ppm) | | | |

### Example 27

43.5 g finely ground crospovidone and 6 g hydroxypropyl cellulose were added to 30 g sodium rabeprazole, mixed well, and then a solution of sodium hydroxide in ethanol (solution of 1.5 g sodium hydroxide dissolved in ethanol) was gradually added to the mixture under stirring to make granules, followed by drying and subsequent regulation of the size of granules in a small type speed mill. 3 % crospovidone and 1.6 % magnesium stearate were added to the regulated granules, mixed and tabletted into tablets each weighing 70 mg containing 10 mg sodium rabeprazole.

### Example 28

The tablets obtained in Example 27 were coated by using a fluidized-layer granulator with a hydrous ethanol solution containing hydroxypropyl cellulose and a slight amount of magnesium stearate, to give tablets having 2 mg intermediate coating laminated thereon. Then, the tablets coated with the intermediate coating were sprayed by using a fluidized-layer granulator with a hydrous ethanol solution containing hydroxypropyl cellulose phthalate, monoglyceride, talc and titanium oxide, to give enteric tablets coated with 10 mg enteric coating.

### Example 29

The enteric tablets obtained in Example 28 were sprayed by using a fluidized-layer granulator with purified water containing hydroxypropylmethyl cellulose, Macrogol 6000^{™} and talc to give tablets coated with 5 mg moisture resistant coating.

## Claims

1. A pharmaceutical composition comprising a core and an enteric coating, wherein the core comprises 1 part by weight of rabeprazole, sodium, 0.5 to 5 parts by weight of crospovidone and 0.01 to 2 parts by weight of sodium hydroxide.

2. The pharmaceutical composition according to claim 1, which further comprises an intermediate coating between the core and the enteric coating, wherein the intermediate coating comprises a water-soluble polymer, a water-soluble or water-disintegrating substance or a water-insoluble substance.

3. The pharmaceutical composition according to claim 1 or 2, which further comprises a moisture resistant coating on the enteric coating, wherein the moisture resistant coating either is a water-soluble coating or a water-insoluble coating.

4. The pharmaceutical composition according to anyone of claims 1 to 3, wherein the core further comprises an antioxidant.

## Patentansprüche

1. Pharmazeutische Zusammensetzung, die einen Kern und eine Magensaft-resistente Beschichtung umfasst, worin der Kern 1 Gew.-Teil Rabeprazol-Natrium, 0,5 bis 5 Gew.-Teile Crospovidon und 0,01 bis 2 Gew.-Teile Natriumhydroxid umfasst.

2. Pharmazeutische Zusammensetzung gemäß Anspruch 1, die weiterhin eine Zwischenbeschichtung zwischen dem Kern und der Magensaft-resistenten Beschichtung umfasst, worin die Zwischenbeschichtung ein wasserlösliches Polymer, eine wasserlösliche oder sich in Wasser zersetzende Substanz oder eine wasserunlösliche Substanz umfasst.

3. Pharmazeutische Zusammensetzung gemäß Anspruch 1 oder 2, die weiterhin auf der Magensaft-resistenten Beschichtung eine Feuchtigkeits-resistente Beschichtung umfasst, worin die Feuchtigkeits-resistente Beschichtung entweder eine wasserlösliche Beschichtung oder eine wasserunlösliche Beschichtung ist.

4. Pharmazeutische Zusammensetzung gemäß mindestens einem der Ansprüche 1 bis 3, worin der Kern weiterhin ein Antioxidans umfasst.

## Revendications

1. Composition pharmaceutique comprenant un noyau et un enrobage entérique, dans laquelle le noyau comprend 1 partie en poids de rabéprazole sodique, de 0,5 à 5 parties en poids de crospovidone et de 0,01 à 2 parties en poids d'hydroxyde de sodium.

2. Composition pharmaceutique selon la revendication 1, qui comprend en outre une couche intermédiaire entre le noyau et l'enrobage entérique, dans laquelle la couche intermédiaire comprend un polymère soluble dans l'eau, une substance soluble dans l'eau ou se désintégrant dans l'eau ou une substance insoluble dans l'eau.

3. Composition pharmaceutique selon la revendication 1 ou 2, qui comprend en outre une couche résistante à l'humidité sur l'enrobage entérique, dans laquelle la couche résistante à l'humidité est soit un enrobage soluble dans l'eau soit un enrobage insoluble dans l'eau.

4. Composition pharmaceutique selon l'une quelconque des revendications 1 à 3, dans laquelle le noyau comprend en outre un antioxydant.
